# EUROPÄISCHE PATENTANMELDUNG

(11) **EP 4 817 638 A1**
(43) Veröffentlichungstag der Anmeldung: **07.10.2026**
(21) Anmeldenummer: 26167872.6
(22) Anmeldetag: 26.03.2026
(51) Int. Cl.: A61L 2/082, A61B 5/153, A61L 2/186, A61L 11/00, B02C 18/00, B09B 3/00, B29B 17/02

(54) **VERFAHREN ZUR WIEDERVERWERTUNG VON MEDIZINISCHEN KUNSTSTOFFABFÄLLEN AUS BLUTENTNAHMERÖHRCHEN**

(30) Priorität: 31.03.2025 AT 502212025
(71) Anmelder: Greiner Bio-One GmbH, 4550 Kremsmünster (AT)
(72) Erfinder: Holzinger, Peter, 4600 Thalheim bei Wels (AT); Bobek, Michael, 4893 Zell am Moos (AT); Fessl, Peter, 4582 Spital am Pyhrn (AT); Mizelli, Maximilian, 4551 Ried im Traunkreis (AT); Niederreiter, Markus, 4522 Sierning (AT)
(74) Vertreter: Burger, Hannes Alfred

(57) **Zusammenfassung**

Die Erfindung betrifft ein Verfahren zur Wiederverwertung von medizinischen Kunststoffabfällen aus Blutentnahmeröhrchen, insbesondere aus Blutentnahmeröhrchen aus PET mit einem darin enthaltenen Trenngel, in einem Recyclingprozess, umfassend das Sammeln der Blutentnahmeröhrchen in einem dafür vorgesehenen Sammelcontainer und das Zerkleinern der Blutentnahmeröhrchen zu Kunststoffabfall oder alternatives Bereitstellen von vorsortiertem Kunststoffabfall durch ein Trennverfahren für Probenaufnahmevorrichtungen, die ein Blutentnahmeröhrchen und eine Verschlusskappe umfassen, sowie ein erstes mikrobiologisches Aufreinigen des vorsortierten Kunststoffabfalls durch chemische Behandlung mit Peressigsäure oder einem anderen Desinfektionsmittel mit antimikrobiologischer Wirkung, sowie ein zweites mikrobiologisches Aufreinigen durch ionisierende Strahlung, bevorzugt Röntgenbestrahlung, zur viralen Sicherheit, sowie ein Entfernen eines Trenngels aus dem Kunststoffabfall durch eine Kombination aus Zentrifugation, Waschung und Erwärmung des Kunststoffabfalls, sowie ein optionales Separieren von Etiketten und Kunststoffverunreinigungen mittels eines Flotationsverfahrens mit einem Trennmedium, sowie eine Trocknung und Regranulierung der Kunststoffabfälle zu einem homogenisierten, recycelten Kunststoffgranulat.

## Beschreibung

Die vorliegende Erfindung betrifft ein Verfahren zur Wiederverwertung von medizinischen Kunststoffabfällen aus Blutentnahmeröhrchen, die aus Polyethylenterephthalat (PET) bestehen.

Der Stand der Technik beschreibt bereits Verfahren zur Wiederaufbereitung von low-density Kunststoffen (PP & PE) aus kontaminierten Krankenhausabfällen, wie beispielsweise in US20110068036A1 und verwandten Dokumenten. Diese bekannten Verfahren basieren auf komplexen Trenn- und Reinigungsprozessen, um niedrigdichte Kunststoffe für die Herstellung neuer Medizinprodukte wiederverwendbar zu machen. Für high-density Kunststoffe wie PET sind diese Verfahren jedoch nicht geeignet, insbesondere wenn die Kunststoffe in Blutentnahmeröhrchen mit Trenngel verwendet wurden.

Ein wesentliches Problem besteht darin, dass das Trenngel an den Kunststoffoberflächen haftet und durch herkömmliche Reinigungsprozesse nur unzureichend entfernt werden kann. Zudem sind die bisher bekannten Sterilisationsmethoden nicht ausreichend, um eine vollständige Entfernung von Viren, Bakterien, Pilzen, Hefe, Prionen und /oder anderweitigen Verunreinigungen zu gewährleisten, insbesondere wenn diese im Trenngel eingeschlossen sind. Diese Problematik wird durch die Zentrifugation von Blutproben in den Röhrchen noch verstärkt, da hierbei virale Partikel in der Gelphase zurückgehalten und während der Dekontamination abgeschirmt bleiben.

Andere bekannte Verfahren, wie beispielsweise aus EP4431195 bekannt geworden, bieten zwar chemische Dekontaminationsmethoden zur Reduktion von Keimen, Sporen und Viren, gewährleisten jedoch keine vollständige virale Sicherheit für die Wiederverwendung des Materials in medizinischen Anwendungen. Darüber hinaus sind diese Verfahren aufgrund aufwendiger Trenn-, Sortier- und Aufbereitungsprozesse wirtschaftlich schwer umsetzbar, da die Qualität des recycelten Materials für eine Wiederverwendung in Medizinprodukten nicht ausreichend sichergestellt werden kann. Ein weiteres Problem besteht in der abfallrechtlichen Einstufung, da die Reinheit des recycelten Materials maßgeblich von der Vermeidung von Fremdstoffen und der vollständigen Entfernung biologischer Rückstände abhängt.

Die Aufgabe der vorliegenden Erfindung besteht darin, die genannten Nachteile des Standes der Technik zu überwinden und ein effizientes Verfahren zur Wiederverwertung von Blutentnahmeröhrchen aus PET bereitzustellen.

Insbesondere soll das Verfahren eine sortenreine Sammlung, eine optimierte Trennung und Reinigung sowie eine zweistufige mikrobiologische Dekontamination ermöglichen. Hierdurch soll sichergestellt werden, dass das recycelte PET-Material frei von viralen und mikrobiologischen Verunreinigungen ist und direkt für die Herstellung neuer Blutentnahmeröhrchen verwendet werden kann.

Das erfindungsgemäße Verfahren bietet eine Reihe von Verbesserungen gegenüber dem Stand der Technik. Durch eine Kombination aus Waschschritten bei höherer Temperatur mit Abtrennung durch Zentrifugation und thermischer Behandlung wird eine effektive Entfernung von Trenngel-Rückständen sichergestellt, wodurch Verunreinigungen reduziert und die Materialreinheit erhöht werden. Die virale und mikrobiologische Sicherheit wird durch eine zweistufige Dekontamination gewährleistet, bestehend aus einer chemischen Behandlung, beispielsweise mit Peressigsäure, und einer physikalischen Sterilisation, bevorzugt mittels Röntgenbestrahlung. Zudem ermöglicht das Verfahren eine digitale Rückverfolgbarkeit des gesamten Recyclingprozesses, wodurch die Qualität des recycelten PET-Materials sichergestellt und die Einhaltung regulatorischer Anforderungen nachgewiesen werden kann. Schließlich zeichnet sich das Verfahren durch eine wirtschaftliche Skalierbarkeit aus, die eine industrielle Umsetzung unter praxisnahen Bedingungen erlaubt und so eine nachhaltige Wiederverwertung von medizinischen Kunststoffen ermöglicht.

Die vorhergehend definierte Aufgabe der Erfindung wird durch ein Verfahren gemäß den Ansprüchen gelöst.

Das erfindungsgemäße Verfahren zur Wiederverwertung von medizinischen Kunststoffabfällen aus Blutentnahmeröhrchen, insbesondere Blutentnahmeröhrchen aus PET mit einem darin enthaltenen Trenngel, in einem Recyclingprozess, umfasst die Schritte:
- sammeln der Blutentnahmeröhrchen in einem dafür vorgesehenen Sammelcontainer und zerkleinern der Blutentnahmeröhrchen zu Kunststoffabfall oder alternatives Bereitstellen von vorsortiertem Kunststoffabfall durch ein Trennverfahren für Probenaufnahmevorrichtungen jeweils umfassend ein Blutentnahmeröhrchen und eine Verschlusskappe;
- erste mikrobiologische Aufreinigung des vorsortierten Kunststoffabfalls durch chemische Behandlung mit Peressigsäure oder einem anderen Desinfektionsmittel mit antimikrobiologischer Wirkung;

- zweite mikrobiologische Aufreinigung durch ionisierende Strahlung, bevorzugt Röntgenbestrahlung, zur viralen Sicherheit;
- Entfernung eines Trenngels aus dem Kunststoffabfall durch eine Kombination aus Zentrifugation, Waschung und Erwärmung des Kunststoffabfalls;
- optionales Separierung von Etiketten und Kunststoffverunreinigungen mittels eines Flotationsverfahrens mit einem Trennmedium;
- Trocknung und Regranulierung der Kunststoffabfälle zu einem homogenisierten, recycelten Kunststoffgranulat für die Herstellung neuer Medizinprodukte, Verpackungen, insbesondere Verpackungen mit hohen Anforderungen bezüglich der mikrobiologische Reinheit deren Ausgangsmaterialien, im Speziellen Pharmaverpackungen oder Blutentnahmeröhrchen.

Als alternative Ausgestaltung des erfindungsgemäßen Verfahrens kann auch vorgesehen sein, dass die gesammelten Blutentnahmeröhrchen erst bei und/oder während der ersten mikrobiologischen Aufreinigung zerkleinert werden.

Jedenfalls werden die Blutentnahmeröhrchen vorzugsweise sortenrein gesammelt. Diesbezüglich kann vorgesehen sein, dass sich die Sortenreinheit auf das Sammeln von ausschließlich Röhrchen, die zur Blutentnahme vorgesehen sind oder waren bezieht und/oder dass sich die Sortenreinheit auf das Sammeln von Röhrchen aus einem bevorzugten Material wie beispielsweise PET bezieht. Insbesondere sind beide Kriterien zu bevorzugen, sodass vorzugsweise Röhrchen aus PET, die ausschließlich zur Blutentnahme vorgesehen sind oder waren, gesammelt werden.

In diesem Zusammenhang kann es auch vorteilhaft sein, dass durch das Verfahren Blutentnahmeröhrchen rezykliert werden, die bereits aus rezykliertem Material ausgebildet sind oder die wenigstens teilweise aus rezykliertem Material ausgebildet sind.

Unter einem Flotationsverfahren ist im Sinne der Erfindung ein physikalisch-chemisches Trennverfahren zu verstehen, das auf den unterschiedlichen Dichte- und Benetzungseigenschaften von Stoffen in einem Flüssigkeitsmedium basiert. Es wird insbesondere zur Separierung von Kunststoffen mit unterschiedlichen Dichten sowie zur Entfernung von anhaftenden Verunreinigungen wie Etiketten, Klebstoffresten oder anderen Störstoffen aus Recyclingströmen eingesetzt. In der Kunststoffaufbereitung kommt das Verfahren insbesondere bei der Wiederverwertung von PET zum Einsatz, da hiermit leichtere Fremdstoffe, wie Etiketten oder Polyolefin-Rückstände, effizient abgetrennt werden können.

Das Grundprinzip der Flotation besteht darin, dass das zu trennende Material in einem Wasserbad oder einer speziell präparierten Flüssigkeit suspendiert wird. Materialien mit einer geringeren Dichte als die Flüssigkeit steigen aufgrund des Auftriebs an die Oberfläche und können dort abgeschöpft oder abgesaugt werden, während dichtere Fraktionen absinken und am Boden separiert werden. Bei der Trennung von Kunststofffraktionen bedeutet dies beispielsweise, dass leichte Polymere wie Polyethylen (PE) oder Polypropylen (PP) aufschwimmen, während schwerere Materialien wie Polyethylenterephthalat (PET) absinken.

Um die Effizienz des Verfahrens zu verbessern, kann die Oberflächenspannung der Flüssigkeit durch Zugabe von Tensiden oder anderen Additiven modifiziert werden. Diese Zusätze reduzieren die Adhäsionskräfte zwischen den Partikeln und der Flüssigkeit, wodurch sich anhaftende Störstoffe wie Papieretiketten oder Klebstoffreste leichter von der Kunststoffoberfläche lösen und abgeschieden werden können. Darüber hinaus kann das Verfahren durch mechanische Unterstützung, wie Rührwerke oder Luftinjektoren, optimiert werden, die eine bessere Durchmischung und eine gleichmäßige Benetzung der Partikel ermöglichen.

Die Anwendung des Flotationsverfahrens in der Wiederverwertung von Blutentnahmeröhrchen aus PET ermöglicht eine gezielte Abtrennung von Etiketten, Transportverpackungen und niederenergetischen Kunststoffanteilen, die für die Materialqualität nachteilig sind. Durch die Entfernung dieser Störstoffe wird sichergestellt, dass das recycelte PET eine hohe Reinheit aufweist und für die Herstellung neuer Medizinprodukte geeignet ist. Da das Verfahren ohne den Einsatz aggressiver Chemikalien auskommt, stellt es zudem eine umweltfreundliche Alternative zur mechanischen oder chemischen Trennung dar und trägt zur nachhaltigen Wiederverwertung medizinischer Kunststoffe bei.

Die erste mikrobiologische Aufreinigung dient der Reduktion von mikrobiellen Verunreinigungen, die sich auf den Oberflächen der recycelten Blutentnahmeröhrchen und deren Komponenten befinden. Dieser Reinigungsschritt erfolgt bevorzugt durch chemische Desinfektionsmittel, die eine breite Wirkung gegen Bakterien, Viren, Sporen und andere pathogene Mikroorganismen aufweisen. Besonders geeignet sind oxidative Desinfektionsmittel, wie Peressigsäure, Wasserstoffperoxid oder Chlordioxid, da sie eine effektive Abtötung von Mikroorganismen bei gleichzeitig geringer Materialbelastung gewährleisten.

Während dieses Prozesses werden die zu reinigenden Kunststoffteile in einem speziell angepassten chemischen Reinigungsbad behandelt. Die Auswahl der Desinfektionslösung sowie deren Konzentration und Einwirkzeit hängt von verschiedenen Faktoren ab, darunter der Art der Mikroorganismen, die entfernt werden sollen, sowie der Beständigkeit der Kunststoffmaterialien gegenüber chemischen Einwirkungen. Durch eine präzise Prozessüberwachung und Echtzeit-Datenanalyse wird sichergestellt, dass die mikrobiologische Reduktion den vorgeschriebenen Sicherheitsstandards entspricht. In diesem Schritt werden zudem häufig anhaftende Etikettenreste oder organische Rückstände aus dem klinischen Umfeld entfernt, wodurch eine erste grobe Reinigung des Materials erfolgt.

Die zweite mikrobiologische Aufreinigung stellt eine physikalische Dekontamination dar, die eine zusätzliche Sicherheitsstufe für die Wiederverwertung des Kunststoffs bietet. Da sich in Blutentnahmeröhrchen enthaltene biologische Verunreinigungen, insbesondere Viren, in Trenngelen oder Polymerstrukturen eingeschlossen sein können, ist eine alleinige chemische Behandlung nicht ausreichend, um eine vollständige mikrobiologische Sicherheit zu gewährleisten. Daher kommt in diesem zweiten Schritt bevorzugt eine ionisierende Strahlung zum Einsatz.

Die Sterilisation erfolgt mittels hochenergetischer Strahlung, bevorzugt durch Röntgenstrahlung, Gamma-Strahlung oder Elektronenstrahlbehandlung. Diese Methoden bewirken eine Zerstörung der DNA und RNA von Mikroorganismen, wodurch Viren, Bakterien und andere pathogene Erreger vollständig inaktiviert werden. Die Wahl der geeigneten Strahlendosis hängt von der Art der Kontaminanten sowie der Materialbeschaffenheit des Kunststoffs ab. Röntgenstrahlung wird dabei bevorzugt eingesetzt, da sie eine gleichmäßige Durchdringung des Materials bei hoher Dosishomogenität ermöglicht, während Gamma- oder Elektronenstrahlung je nach Dichte des Materials unterschiedlich tief eindringen kann.

Zusätzlich zu ihrer mikrobiellen Wirkung ermöglicht die zweite mikrobiologische Aufreinigung eine Verbesserung der Materialqualität, indem restliche flüchtige organische Substanzen und andere unerwünschte Rückstände entfernt werden. Dies trägt zur Stabilisierung des Recyclingprozesses bei und stellt sicher, dass das recycelte PET-Material den strengen regulatorischen Anforderungen für medizinische Anwendungen entspricht.

Durch die Kombination dieser beiden mikrobiologischen Aufreinigungsverfahren bzw. - schritte wird ein hocheffizientes und sicheres Recyclingverfahren geschaffen, das die kontaminationsfreie Wiederverwertung von Blutentnahmeröhrchen aus PET ermöglicht. Während die erste Reinigung eine grundlegende mikrobiologische Dekontamination und Entfernung von Oberflächenverunreinigungen bewirkt, sorgt die zweite Stufe für eine tiefgehende mikrobiologische Sicherheit durch physikalische Sterilisation. Dieses zweistufige Verfahren gewährleistet, dass das recycelte Material für eine erneute Verwendung in hochsensiblen medizinischen Produkten geeignet ist.

Ein zentrales Problem beim Recycling von Blutentnahmeröhrchen aus PET ist das im Inneren enthaltene Trenngel, das während der Laboranalyse zur Phasentrennung von Blutbestandteilen eingesetzt wird. Dieses Gel besteht aus hochviskosen, hydrophoben Polymermischungen, die eine starke Adhäsion an den Kunststoffwänden des Röhrchens aufweisen. Eine vollständige Entfernung des Trenngels ist essenziell, da es Verunreinigungen im recycelten Material verursachen und dessen mechanische und chemische Eigenschaften negativ beeinflussen kann.

Die Entfernung des Trenngels erfolgt in einem zweistufigen Prozess, der Zentrifugation mit Waschung und Erwärmung kombiniert, um eine möglichst vollständige Abtrennung des Gels zu gewährleisten. Zunächst werden die gesammelten Röhrchen einer mechanischen Vorbehandlung unterzogen, bei der grobe Rückstände des Gels durch einen ersten Waschvorgang mit wässrigen oder alkoholbasierten Reinigungsmitteln gelöst werden. Anschließend folgt die Zentrifugationsstufe, in der das Trenngel durch eine hohe Rotationskraft von den Röhrchenwänden gelöst und mechanisch unter Zuhilfenahme der Erwärmung durch die Waschlösung abgeschieden wird.

Da das Trenngel aufgrund seiner tixotropen Eigenschaften unter normalen Bedingungen eine hohe Viskosität aufweist, ist eine thermische Unterstützung erforderlich, um den Prozess zu optimieren. Durch eine gezielte Erwärmung des Materials auf Temperaturen von wenigstens 50°C wird das Gel verflüssigt, wodurch es sich leichter von der PET-Oberfläche ablösen lässt. Dieser Temperaturbereich ist so gewählt, dass das Trenngel seine Fließeigenschaften verbessert, ohne jedoch das Kunststoffmaterial der Röhrchen thermisch zu schädigen.

Nach der Zentrifugation und Erwärmung erfolgt eine abschließende Hochtemperatur-Wäsche, um letzte Gelreste sowie eventuell im Gel eingeschlossene biologische Kontaminationen zu entfernen. Hierbei können zusätzlich Tenside oder enzymatische Reinigungsmittel eingesetzt werden, um verbleibende Rückstände effektiv zu lösen. Dieser Schritt stellt sicher, dass das PET-Material frei von gelartigen Verunreinigungen ist und für die nachfolgenden mikrobiologischen Reinigungs- und Recyclingprozesse in einer optimalen Qualität vorliegt.

Durch die Kombination von Zentrifugation und Erwärmung wird eine hocheffiziente und materialschonende Entfernung des Trenngels ermöglicht. Dieses Verfahren gewährleistet, dass die Röhrchen für den weiteren Recyclingprozess in einer sortenreinen und homogenen Form bereitgestellt werden und keine Störstoffe im späteren Kunststoffgranulat verbleiben. Damit wird nicht nur die Qualität des recycelten PET verbessert, sondern auch sichergestellt, dass die daraus hergestellten Produkte höchsten hygienischen und regulatorischen Anforderungen genügen.

Das erfindungsgemäße Verfahren bietet eine effiziente, sichere und industriell umsetzbare Lösung zur Wiederverwertung von Blutentnahmeröhrchen aus PET, die bisher aufgrund ihrer spezifischen Verunreinigungen, insbesondere durch Trenngel und biologische Kontaminationen, nur schwer recycelbar waren. Durch die sortenreine Sammlung in spezialisierten Sammelcontainern wird sichergestellt, dass die Materialströme frei von Fremdstoffen und Vermischungen mit anderen Kunststoffabfällen bleiben, wodurch die Qualität des recycelten PET erheblich gesteigert wird. Die digitale Rückverfolgbarkeit des gesamten Recyclingprozesses ermöglicht eine lückenlose Dokumentation der Herkunft, Behandlung und Reinheit des Materials, was nicht nur regulatorische Anforderungen erleichtert, sondern auch eine transparente Überwachung und Qualitätskontrolle in der gesamten Prozesskette gewährleistet.

Ein wesentliches Alleinstellungsmerkmal des Verfahrens ist die zweistufige mikrobiologische Reinigung, die sowohl eine chemische Desinfektion mit Peressigsäure als auch eine physikalische Sterilisation durch Röntgenbestrahlung umfasst. Diese Kombination stellt sicher, dass sowohl oberflächliche als auch tief eingebettete biologische Kontaminationen, einschließlich Viren, Bakterien und Sporen, vollständig entfernt werden, wodurch das recycelte Material wieder für den Einsatz in medizinischen Anwendungen geeignet wird. Besonders vorteilhaft ist dabei, dass die ionisierende Strahlung eine gleichmäßige Durchdringung des Materials ermöglicht, ohne dessen mechanische Eigenschaften zu beeinträchtigen.

Ein weiteres zentrales Merkmal ist die gezielte Entfernung des Trenngels, das durch Zentrifugation und Erwärmung gelöst wird. Diese Methode gewährleistet eine effektive und materialschonende Abtrennung des Gels, ohne dass es sich mit den Kunststofffragmenten vermischt oder im nachfolgenden Recyclingprozess zu Störungen führt. Die nachfolgende Flotation und/oder eine Windsichtung zur Separierung von Etiketten und leichten Kunststoffverunreinigungen verbessert die Materialreinheit zusätzlich, indem störende Bestandteile effizient entfernt werden, bevor das Material weiterverarbeitet wird.

Durch die abschließende Trocknung und Regranulierung wird das gereinigte PET in eine homogene Rohstoffqualität überführt, die vergleichbare Eigenschaften wie neu hergestelltes PET aufweist. Dies ermöglicht eine direkte Wiederverwendung in der Herstellung neuer Medizinprodukte, insbesondere Blutentnahmeröhrchen, wodurch nicht nur der Einsatz von Neuware reduziert, sondern auch die Nachhaltigkeit und Kreislauffähigkeit medizinischer Kunststoffe erheblich verbessert wird. Das Verfahren stellt somit einen wirtschaftlich tragfähigen, umweltfreundlichen und regulatorisch konformen Recyclingprozess bereit, der sowohl Abfallmengen reduziert als auch die Versorgung mit hochwertigen, wiederverwerteten Rohstoffen für den medizinischen Sektor sichert.

Das Trennverfahren zur Bereitstellung von vorsortiertem Kunststoffmaterial basiert auf einer mehrstufigen Recyclingstrategie, um die verschiedenen Materialkomponenten effizient zu separieren und sortenrein wiederaufzubereiten. In der ersten Stufe wird eine Probenaufnahmevorrichtung, die ein Blutentnahmeröhrchen und eine dafür vorgesehene Verschlusskappe bzw. Kappe umfasst, geschreddert bzw. zerkleinert, gewaschen und dekontaminiert bzw. vorzugsweise durch Gammastrahlung behandelt, um organische Rückstände und biologische Kontaminationen zu entfernen. Dabei kommt eine chemische Reinigung mit Peressigsäure (PAA, z. B. Proxitane 14:23) zum Einsatz, um eine mikrobiologische Sicherheit zu gewährleisten. Dies stellt sicher, dass sämtliche Blut- oder Gelreste entfernt werden, bevor das Material einer weiteren Verarbeitung unterzogen wird.

In der zweiten Stufe wird das gereinigte, zerkleinerte Material einem Float-Sink-Verfahren unterzogen, um die verschiedenen Kunststoffbestandteile anhand ihrer Dichteunterschiede zu trennen. Das PET-Material der Röhrchen besitzt eine höhere Dichte von etwa 1,2-1,3 g/cm³, während leichtere Fremdstoffe, wie Schrumpffolien aus LDPE oder der Dichtstopfen, eine Dichte von ca. 0,91-0,93 g/cm³ aufweisen. Diese Trennung erfolgt in einem Wasserbad oder einem anderen definierten Flüssigkeitsmedium mit einer gezielt eingestellten Dichte. Während PET-Partikel aufgrund ihrer höheren Dichte absinken, schwimmen leichtere Kunststoffbestandteile an der Oberfläche und können dort abgeschöpft werden. Dieses Verfahren ermöglicht eine effiziente Separierung von Fremdstoffen, um eine hochwertige und sortenreine Wiederaufbereitung des PET-Materials sicherzustellen.

Im Zusammenhang mit erfindungsgemäßem Verfahren kann es weiters zweckmäßig sein, wenn das Verfahren den Schritt einer digitalen Rückverfolgbarkeit des Recyclingprozesses durch Codierung des Sammelcontainers und Verknüpfung der Codierung mit einem digitalen System umfasst, wobei das recycelte Kunststoffgranulat zur Herstellung neuer Blutentnahmeröhrchen mit, im digitalen System dokumentierter Reinheit und nachvollziehbarem Recycling verwendet wird.

Unter einer digitalen Codierung und deren Verknüpfung zu einem digitalen System ist im Sinne der Erfindung ein System zur Rückverfolgbarkeit und Qualitätskontrolle des Recyclingprozesses für medizinische Kunststoffabfälle zu verstehen. Diese Technologie ermöglicht eine lückenlose Dokumentation des Materialflusses, angefangen bei der Sammlung der Abfälle in medizinischen Einrichtungen bis hin zur Verarbeitung, Reinigung und Wiederverwertung der recycelten Kunststoffe. Durch die digitale Erfassung jedes Prozessschrittes kann sichergestellt werden, dass das recycelte Material den hygienischen und regulatorischen Anforderungen entspricht und zuverlässig für die Herstellung neuer Medizinprodukte verwendet werden kann.

Die digitale Codierung erfolgt in der Regel durch die Kennzeichnung der Sammelbehälter oder einzelnen Materialchargen mit einem eindeutigen Identifikationsmerkmal. Dies kann in Form eines Barcode- oder QR-Code-Systems, durch RFID-Transponder oder mittels einer Blockchain-basierten Codierung erfolgen. Durch die Verknüpfung dieser Codes mit einer zentralen Datenbank oder einem vernetzten ERP-System kann der gesamte Materialstrom in Echtzeit überwacht und dokumentiert werden. Dies ermöglicht es, kritische Parameter wie Herkunft, Sterilisationsschritte, chemische Behandlungen und physikalische Prozesse zu protokollieren, um eine vollständige Transparenz entlang der gesamten Recyclingkette zu gewährleisten.

Die Verknüpfung der digitalen Codierung mit einem digitalen System ermöglicht zusätzlich eine automatisierte Qualitätskontrolle. Beispielsweise können Messdaten aus chemischen Reinigungsprozessen oder Sterilisationsschritten direkt mit der Materialcharge verknüpft werden. Dies erlaubt eine automatisierte Verifikation der mikrobiellen Reduktion, sodass nur PET-Materialien weiterverarbeitet werden, die die erforderlichen Reinheitsstandards erfüllen. Auch die Identifikation von Chargen mit Abweichungen oder potenziellen Verunreinigungen kann in Echtzeit erfolgen, wodurch Ausschuss reduziert und die Prozesssicherheit erhöht wird.

Ein weiterer Vorteil der digitalen Verknüpfung besteht in der Integration mit regulatorischen Anforderungen und der Möglichkeit, relevante Nachweise für Behörden und Kunden bereitzustellen. In einem Blockchain-basierten System könnte beispielsweise die gesamte historische Prozesskette einer Charge unveränderlich gespeichert werden, sodass im Falle einer behördlichen Prüfung oder eines Auditverfahrens eine vollständige Dokumentation vorliegt. Diese Transparenz stärkt nicht nur das Vertrauen in das recycelte Material, sondern ermöglicht auch eine vereinfachte Zertifizierung und Zulassung der daraus hergestellten Produkte für den medizinischen Einsatz.

Zusammenfassend ermöglicht die digitale Codierung in Verbindung mit einem digitalen System eine effiziente, sichere und rückverfolgbare Wiederverwertung medizinischer Kunststoffe. Sie stellt sicher, dass nur qualitativ hochwertiges und mikrobiologisch einwandfreies Material für die Produktion neuer Medizinprodukte genutzt wird, verbessert die Prozessautomatisierung und trägt zur rechtlichen und umwelttechnischen Compliance bei.

Die Verwendung des recycelten Kunststoffgranulats zur Herstellung neuer Blutentnahmeröhrchen mit im digitalen System dokumentierter Reinheit und nachvollziehbarem Recycling bietet erhebliche Vorteile in Bezug auf Qualitätskontrolle, Transparenz und regulatorische Compliance. Durch die digitale Dokumentation jedes Prozessschritts kann sichergestellt werden, dass das recycelte Material nachweislich frei von biologischen und chemischen Verunreinigungen ist und den hohen Anforderungen für den medizinischen Einsatz entspricht. Zudem ermöglicht die Rückverfolgbarkeit eine klare Trennung zwischen Materialchargen, wodurch Fehler und Vermischungen ausgeschlossen werden. Dies schafft nicht nur Vertrauen in die Wiederverwendung des Materials, sondern erleichtert auch Zertifizierungs- und Zulassungsverfahren für die daraus hergestellten Medizinprodukte.

Ferner kann vorgesehen sein, dass die Sammlung der Röhrchen mittels dem Sammelcontainer mit automatisierter Entkappung und Sortierung der Blutentnahmeröhrchen erfolgt und der Sammelcontainer mit einem Originalitätsverschluss versehen ist.

Die automatisierte Entkappung und Sortierung der Blutentnahmeröhrchen im Sammelcontainer verbessert die Effizienz und Reinheit des Recyclingprozesses, da die Röhrchen bereits in einem standardisierten Verfahren vorbereitet und sortiert werden, bevor sie weiterverarbeitet werden. Dies reduziert den manuellen Aufwand und verringert das Risiko von Fehlbefüllungen oder Verunreinigungen innerhalb des Sammelbehälters. Der Originalitätsverschluss sorgt zusätzlich dafür, dass der Container nach der Befüllung nicht unbefugt geöffnet oder manipuliert werden kann, wodurch die Sicherheit des gesammelten Materials gewährleistet wird.

Im diesem Sinne ist unter einem Originalitätsverschluss eine sicherheitsrelevante Versiegelung eines Behälters zu verstehen, die nach dem ersten Verschließen nicht ohne sichtbare Spuren geöffnet werden kann. Solche Verschlüsse sind in der Verpackungsindustrie weit verbreitet und werden beispielsweise in der Lebensmittel- und Pharmaindustrie verwendet, um sicherzustellen, dass ein Produkt ungeöffnet und unverändert beim Verbraucher oder in der Weiterverarbeitung ankommt. Im vorliegenden Verfahren dient der Originalitätsverschluss dazu, zu verhindern, dass gesammelte Blutentnahmeröhrchen nachträglich verunreinigt oder durch andere Abfälle vermischt werden, was die Prozesssicherheit und Materialreinheit erheblich verbessert.

Darüber hinaus kann vorgesehen sein, dass die ionisierende Strahlung mit einer Dosis zwischen 10 kGy und 50 kGy durchgeführt wird, um DNA- und RNA-basierte Kontaminationen zu eliminieren.

Die Durchführung der ionisierenden Strahlung mit einer Dosis zwischen 10 kGy und 50 kGy gewährleistet eine effektive Eliminierung von DNA- und RNA-basierten Kontaminationen, insbesondere von Viren, Bakterien und Sporen. Diese Strahlendosis wurde gezielt so gewählt, dass sie eine maximale Dekontamination sicherstellt, ohne das recycelte PET-Material in seinen mechanischen oder chemischen Eigenschaften zu beeinträchtigen. Im Vergleich zu herkömmlichen chemischen Sterilisationsmethoden bietet die ionisierende Strahlung den Vorteil, dass sie eine gleichmäßige Durchdringung des Materials ermöglicht und damit auch tief im Polymer eingeschlossene biologische Verunreinigungen zerstört. Die Wahl einer definierten Dosis im Bereich von 10 kGy bis 50 kGy stellt sicher, dass sowohl niedrig- als auch hochresistente Mikroorganismen und Viren zuverlässig abgetötet werden, wodurch die Sicherheit des recycelten Materials für die Wiederverwendung in medizinischen Anwendungen gewährleistet wird.

Vorteilhaft ist auch eine Ausprägung, gemäß welcher vorgesehen sein kann, dass das Trenngel durch eine zusätzliche Waschung mit Wasser während der Zentrifugation und bei einer Temperatur aus einem Temperaturbereich umfassend 35°C bis 95°C entfernt wird.

Die zusätzliche Waschung mit Wasser während der Zentrifugation bei einer Temperatur zwischen 35°C und 95°C optimiert die Entfernung des Trenngels erheblich. Das Trenngel weist tixotrope Eigenschaften auf, wodurch es unter normalen Bedingungen eine hohe Viskosität besitzt und schwer zu entfernen ist. Durch die Kombination aus Zentrifugalkraft und erhöhter Temperatur wird das Gel verflüssigt und kann sich leichter von den PET-Röhrchenwänden ablösen. Die zusätzliche Wasserspülung während dieses Prozesses sorgt dafür, dass gelöste Gelreste sofort weggespült werden, wodurch eine Verschleppung oder Wiederverschmutzung der Röhrchen verhindert wird.

Ein weiterer Vorteil der temperaturgesteuerten Waschung besteht darin, dass durch die Wahl eines optimalen Temperaturbereichs von 35°C bis 95°C das Gel zwar weich genug wird, um entfernt zu werden, aber gleichzeitig die thermische Belastung des PET-Materials minimal bleibt. Dies stellt sicher, dass die Röhrchen ihre strukturelle Integrität behalten und sich nicht verformen oder in ihrer chemischen Zusammensetzung verändern. Die Kombination aus mechanischer, thermischer und wasserbasierter Reinigung bietet somit eine effektive, materialschonende und umweltfreundliche Lösung zur Entfernung des Trenngels und verbessert die Reinheit des recycelten PET erheblich.

Gemäß einer Weiterbildung ist es möglich, dass das Floatationsverfahren mit einer Dichteanpassung des Trennmediums erfolgt, um eine verbesserte Separierung von Fremdstoffen zu ermöglichen.

Die Durchführung des Floatationsverfahrens mit einer Dichteanpassung des Trennmediums ermöglicht eine besonders präzise Separierung von Fremdstoffen aus dem zu recycelnden PET-Material. Durch gezielte Anpassung der Dichte des Trennmediums kann eine effektive Trennung unterschiedlicher Kunststoffarten und anderer Verunreinigungen gewährleistet werden. Materialien mit einer Dichte oberhalb der gewählten Trennmediumdichte sinken ab, während leichtere Verunreinigungen aufschwimmen und einfach entfernt werden können. Dadurch wird die Materialreinheit signifikant erhöht, was nicht nur die Qualität des recycelten Kunststoffs verbessert, sondern auch nachgelagerte Aufbereitungsprozesse vereinfacht. Zudem wird die Effizienz des gesamten Recyclingverfahrens gesteigert, da weniger aufwendige mechanische oder chemische Trennschritte erforderlich sind.

Das Floatationsverfahren wird bevorzugt als Float-Sink-Verfahren durchgeführt, bei dem das Trennmedium eine gezielt eingestellte Dichte aufweist, um unterschiedliche Kunststofffraktionen voneinander zu trennen. Die Trennung erfolgt typischerweise in einem Medium mit einer Dichte, die zwischen der Dichte des PET-Materials (ca. 1,2-1,3 g/cm³) und der Dichte von leichteren Fremdstoffen, wie z. B. Schrumpffolien oder Etiketten aus LDPE (ca. 0,91-0,93 g/cm³), liegt. PET-Partikel sinken in diesem Medium ab, während leichtere Kunststoffe und anhaftende Verunreinigungen an der Oberfläche verbleiben und mechanisch abgeschöpft werden können. Durch diese gezielte Trennung wird sichergestellt, dass das gewonnene PET-Granulat eine hohe Reinheit aufweist und für die Herstellung neuer Medizinprodukte geeignet ist.

Als Trennmedien kommen verschiedene Flüssigkeiten infrage, die eine gezielte Dichteanpassung ermöglichen. Besonders geeignet sind Wasser mit zugesetzten Salzen (z. B. Kochsalzlösung mit 15 % NaCl), organische Lösungsmittel mit definierten Dichten oder spezielle wässrige Polymerlösungen, die je nach Konzentration auf bestimmte Dichtewerte eingestellt werden können. Die Dichteanpassung ermöglicht eine maßgeschneiderte Separation, indem sie auf die spezifischen Materialeigenschaften der zu trennenden Stoffe abgestimmt wird. Dies bedeutet, dass die Wahl des Trennmediums so getroffen wird, dass sowohl die zu recycelnde PET-Fraktion zuverlässig separiert als auch störende Bestandteile sicher entfernt werden. Die Flexibilität dieser Methode erlaubt es, je nach Zusammensetzung des Abfallsystems gezielt auf unterschiedliche Verunreinigungen einzugehen und den Prozess an wechselnde Materialströme anzupassen.

Ferner kann es zweckmäßig sein, wenn die Trocknung der Kunststoffabfälle unter Vakuum erfolgt, um Restverunreinigungen zu minimieren.

Die Trocknung der Kunststoffabfälle unter Vakuum bietet den entscheidenden Vorteil, dass Restfeuchtigkeit und flüchtige Verunreinigungen effizient entfernt werden. Durch das reduzierte Druckniveau wird die Verdampfungstemperatur von Wasser und anderen Flüssigkeiten gesenkt, wodurch selbst tief im Material eingeschlossene Feuchtigkeit schneller und bei niedrigeren Temperaturen entweichen kann. Dies ist besonders vorteilhaft für die nachfolgende Verarbeitung, da verbleibende Feuchtigkeit die Qualität der Schmelzverarbeitung negativ beeinflussen und zu Porenbildung oder Hydrolyse des PET führen könnte. Zudem werden durch die Vakuumtrocknung auch leicht flüchtige organische Rückstände, Lösungsmittelreste oder Spuren chemischer Reinigungsmittel eliminiert, wodurch die Materialreinheit und die mechanischen Eigenschaften des recycelten Kunststoffs weiter verbessert werden.

Darüber hinaus kann vorgesehen sein, dass die Regranulierung durch eine Schmelzfiltration mittels eines Filters mit einer Maschenweite unter 50 µm, im Speziellen unter 30 µm, insbesondere unter 20 µm ergänzt wird.

Die Ergänzung der Regranulierung durch eine Schmelzfiltration mit einer Maschenweite unter 50 µm stellt sicher, dass selbst feinste Verunreinigungen, wie ungelöste Partikel, Polymerabbauprodukte oder anorganische Rückstände, aus dem aufgeschmolzenen Kunststoff entfernt werden. Diese Filtrationsstufe ist entscheidend, um eine homogene und hochreine Granulatqualität zu gewährleisten, die mit Neuware vergleichbar ist. Besonders in medizinischen Anwendungen, in denen höchste Materialreinheit gefordert ist, trägt die Schmelzfiltration dazu bei, dass keine Fremdstoffe oder unerwünschte Defekte in den neu hergestellten Blutentnahmeröhrchen auftreten. Zudem verbessert die Entfernung feinster Partikel die Verarbeitungseigenschaften des recycelten PET, wodurch eine gleichmäßige Schmelzviskosität und eine optimierte Formgebung im Spritzguss- oder Extrusionsverfahren erzielt werden.

Des Weiteren kann vorgesehen sein, dass das recycelte Kunststoffgranulat für die erneute Herstellung von Blutentnahmeröhrchen mit einem IV-Wert zwischen 0,55 dl/g und 0,65 dl/g bereitgestellt wird.

Die Bereitstellung des recycelten Kunststoffgranulats mit einem IV-Wert zwischen 0,55 dl/g und 0,65 dl/g stellt sicher, dass das Material für die erneute Herstellung von Blutentnahmeröhrchen optimal geeignet ist. Die intrinsische Viskosität (IV-Wert) ist eine zentrale Kenngröße für die Molekularstruktur von PET und beeinflusst maßgeblich die mechanischen Eigenschaften, Fließfähigkeit und Verarbeitungseigenschaften des Kunststoffs. Ein höherer IV-Wert korreliert mit einer höheren Molmasse des Polymers, was sich positiv auf die mechanische Festigkeit und Zähigkeit des Materials auswirkt. Gleichzeitig kann ein zu hoher IV-Wert die Fließfähigkeit im Schmelzzustand negativ beeinflussen, wodurch die Verarbeitung, insbesondere im Spritzgussverfahren, erschwert wird.

Durch die gezielte Einstellung des IV-Werts im Bereich 0,55-0,65 dl/g wird ein optimaler Kompromiss zwischen mechanischer Stabilität und Verarbeitbarkeit erreicht. Ein IV-Wert im unteren Bereich dieser Spanne (0,55-0,58 dl/g) verbessert die Fließeigenschaften und ermöglicht eine präzisere Formgebung, was besonders bei der Herstellung von dünnwandigen, hochtransparenten Röhrchen von Vorteil ist. Höhere IV-Werte innerhalb des Bereichs (0,60-0,65 dl/g) sorgen hingegen für höhere Festigkeit und Bruchsicherheit, was die mechanische Belastbarkeit der Röhrchen erhöht und deren Einsatzsicherheit im medizinischen Umfeld verbessert.

Ein weiterer Vorteil der definierten IV-Spanne ist die Prozessstabilität im Recyclingkreislauf. Während bei der Wiederverwertung von PET der IV-Wert durch thermische und mechanische Belastung absinken kann, ermöglicht die gezielte Anpassung des IV-Werts vor der Granulierung eine gleichbleibende Qualität der Endprodukte. Dies ist insbesondere bei medizinischen Anwendungen essenziell, da hier präzise Materialeigenschaften erforderlich sind, um Sicherheits- und regulatorische Vorgaben zu erfüllen .

Zusammenfassend stellt die Einstellung des IV-Werts auf 0,55-0,65 dl/g sicher, dass das recycelte PET-Granulat hochwertige, verarbeitungstechnisch optimierte und mechanisch belastbare Blutentnahmeröhrchen ermöglicht. Diese gezielte Kontrolle trägt zur Effizienz und Nachhaltigkeit des Recyclingprozesses bei, indem sie eine gleichbleibende Materialqualität gewährleistet und gleichzeitig eine Wiederverwendung im medizinischen Bereich erlaubt.

Bei der Verarbeitung von recyceltem PET ist die intrinsische Viskosität (IV) ein entscheidender Parameter. Die IV ist ein Maß für die Kettenlänge der Polymermoleküle und beeinflusst maßgeblich die Verarbeitungseigenschaften des Materials. Für PET ist die intrinsische Viskosität besonders relevant, da sie einen Hinweis auf die Molmasse und damit auf die mechanischen und physikalischen Eigenschaften des Materials gibt. Die intrinsische Viskosität von PET kann in verschiedenen Lösungsmitteln und bei unterschiedlichen Temperaturen bestimmt werden. Übliche Lösungsmittel sind zum Beispiel Dichloressigsäure oder Phenol/Tetrachlorethan- Gemische. Die Messung der intrinsischen Viskosität erfolgt typischerweise mithilfe eines Kapillarviskosimeters, wobei die Viskosität der Polymerlösung und des reinen Lösungsmittels bei verschiedenen Polymerkonzentrationen bestimmt wird.

Jedenfalls hat intrinsische Viskosität einen direkten Einfluss auf die Verarbeitbarkeit von PET, indem sie die Fließfähigkeit, die mechanischen Eigenschaften und das Schmelzverhalten des Materials beeinflusst. Hersteller müssen die IV bei der Auswahl des Materials und der Festlegung der Verarbeitungsparameter sorgfältig berücksichtigen, um die gewünschten Produkteigenschaften zu erreichen und die Effizienz des Produktionsprozesses zu maximieren.

Es kann in diesem Zusammenhang insbesondere vorteilhaft sein, wenn ein IV-Wert von 0,58 dl/g oder insbesondere unter 0,58 dl/g, gewählt wird.

Die geringe intrinsische Viskosität des PET-Materials führt unter anderem zu folgenden Vorteilen.

Die niedrigere Viskosität der Polymerschmelze ermöglicht eine bessere Fließfähigkeit beim Spritzgussverfahren. Dies führt zu einer geringeren Röhrchenbiegung, einer verbesserten Oberflächenqualität und einer höheren Maßhaltigkeit der Röhrchen.

Die reduzierte Viskosität ermöglicht eine Senkung der Einspritzdrücke und der erforderlichen Zuhaltekraft der Spritzgussmaschine. Dadurch wird der Energieverbrauch bei der Herstellung der Röhrchen reduziert.

In diesem Zusammenhang ist es weiters noch erwähnenswert, dass das Kunststoffmaterial bei Herstellung des Blutentnahmeröhrchens und/oder bei Herstellung des spritzgussfähigen Granulates des Materials des Blutentnahmeröhrchens gefiltert wird. Standardmäßig kommt dabei für PET ein Siebfilter mit einer Maschenweite von 50 µm bei einer intrinsischen Viskosität von etwa 0,8 dl/g zum Einsatz. Dadurch ergibt sich der Vorteil, dass geringere Maschenweiten von bis etwa 20 µm mit in der Folge verbesserter Filterqualität bei im Wesentlichen gleichen oder ähnlichen Druckverlusten einsetzbar sind. Weiters können somit bei gleicher Maschenweite das Risiko für Materialschädigungen durch einen geringeren Filterdruckaufbau als auch der benötigte Energiebedarf bei der Filterung reduziert werden. Dadurch kann einerseits Kunststoffmaterial mit einem höheren Recyclinganteil bzw. einem niedrigeren Anteil an nicht-recyceltem Kunststoffmaterial eingesetzt werden und andererseits die Qualität des Blutentnahmeröhrchens verbessert oder hinsichtlich dessen Recyclinganteil gleichbehalten werden oder sogar der Energieaufwand für den Herstellungsprozess verringert werden.

Die verbesserte Fließfähigkeit ermöglicht die Herstellung von Röhrchen mit geringeren Wandstärken. Dies ist insbesondere für Anwendungen von Vorteil, bei denen ein geringes Gewicht und eine hohe Transparenz gewünscht sind, wie beispielsweise bei Becheranwendungen oder Preforms für Plastikflaschen.

Die geringere Röhrchenbiegung und die geringeren Wandstärkenunterschiede führen zu einer höheren Qualität der hergestellten Röhrchen.

Ferner kann vorgesehen sein, dass neue Blutentnahmeröhrchen aus dem homogenisierten, recycelten Kunststoffgranulat hergestellt werden.

Erfindungsgemäß kann das, gemäß dem Verfahren entsprechend vorhergehenden Erläuterungen hergestellte homogenisierte, recycelte Kunststoffgranulat zur Herstellung von neuen Medizinprodukten, insbesondere zur Herstellung von Verpackungen mit Anforderungen bezüglich der mikrobiologischen Reinheit deren Ausgangsmaterialien, im Speziellen von Pharmaverpackungen oder von Blutentnahmeröhrchen, verwendet werden.

Vorteilhafterweise kann vorgesehen sein, dass die Herstellung der neuen Medizinprodukte unter Verwendung des recyceltem Kunststoffgranulats mittels eines Co-Injektionsspritzgussverfahrens oder mittels eines Overmolding-Spritzgussverfahrens durchgeführt wird.

Das Co-Injektionsspritzgussverfahren ist eine spezielle Technik des Spritzgießens, bei der zwei verschiedene Kunststoffe in einem einzigen Prozessschritt in eine Form eingespritzt werden. Dabei wird zunächst ein Kernmaterial eingespritzt, das anschließend oder vielmehr gleichzeitig von einer äußeren Kunststoffschicht umhüllt wird. Dieses Verfahren ermöglicht die Kombination unterschiedlicher Materialeigenschaften, wie z. B. hohe Festigkeit im Inneren und eine optisch ansprechende oder widerstandsfähige Oberfläche. Es wird häufig zur Reduzierung von Materialkosten, zur Verbesserung mechanischer Eigenschaften oder zur Erzielung spezifischer Funktionen wie Barriereeigenschaften oder Recyclinganwendungen eingesetzt.

Das Overmolding-Spritzgussverfahren ist eine Technik, bei der ein Grundkörper mit einer zweiten Materialschicht umspritzt wird, um eine dauerhafte Verbindung zwischen beiden Komponenten zu schaffen. Dabei kann das Substrat aus Kunststoff oder denkbar auch aus einem anderen Material bestehen, während die Überformung meist mit einem weicheren oder funktionaleren Kunststoff erfolgt. Dieses Verfahren kann beispielsweise genutzt werden, um rutschfeste Oberflächen oder Abdichtungen zu erzeugen. Overmolding verbessert nicht nur die Haptik und Optik eines Produkts, sondern kann auch dessen mechanische Eigenschaften und Widerstandsfähigkeit gegenüber äußeren Einflüssen steigern, sowie den Einsatz von recyceltem Material ermöglichen.

Die Herstellung neuer Blutentnahmeröhrchen aus recyceltem Kunststoff mittels eines Co-Injektionsspritzgussverfahrens bietet erhebliche Vorteile in Bezug auf Materialeffizienz, Qualität und Nachhaltigkeit. Beim Co-Injektionsspritzgussverfahren wird ein Material mit hohem Recyclinganteil in einer Kernschicht verarbeitet, während eine äußere Schicht aus hochwertigem Neumaterial oder speziell aufbereiteter Recyclat-Schicht das Produkt ummantelt. Dies ermöglicht die Nutzung eines hohen Anteils an recyceltem PET (rPET), ohne Kompromisse bei den mechanischen und hygienischen Eigenschaften der Endprodukte einzugehen.

Ein entscheidender Vorteil ist, dass die äußere Schicht die Barriereeigenschaften und Transparenz des Röhrchens verbessert, während die innere Schicht kosteneffizient aus recyceltem Material bestehen kann. Dies ist besonders für medizinische Anwendungen relevant, da die äußere Schicht direkten Kontakt mit der Umgebung hat und höchste Reinheits- und Sicherheitsstandards erfüllen muss. Gleichzeitig wird durch diese Struktur die mechanische Stabilität des Röhrchens erhöht, da die Kombination aus recyceltem PET und Neumaterial eine optimierte Balance zwischen Festigkeit und Flexibilität bietet.

Ein weiterer bedeutender Vorteil des Co-Injektionsspritzgusses liegt in der Möglichkeit, gezielt funktionale Schichten zu integrieren, die beispielsweise eine erhöhte chemische Beständigkeit oder optimierte Barriereeigenschaften gegen Feuchtigkeit oder Gase bieten können. Zudem erlaubt das Verfahren eine gleichmäßige Verteilung der Materialeigenschaften über das gesamte Röhrchen, wodurch unerwünschte Materialschwankungen reduziert und eine gleichbleibend hohe Produktqualität sichergestellt wird.

Zusammenfassend trägt der Einsatz des Co-Injektionsspritzgussverfahrens wesentlich dazu bei, den Anteil an recyceltem Material in medizinischen Kunststoffprodukten zu maximieren, ohne dabei Abstriche bei Sicherheit, Transparenz oder mechanischer Festigkeit zu machen. Dies ermöglicht nicht nur eine nachhaltigere Kreislaufwirtschaft, sondern reduziert auch die Abhängigkeit von neuem PET, was sowohl ökologische als auch wirtschaftliche Vorteile mit sich bringt.

Erfindungsgemäß ist ein Blutentnahmeröhrchen zur Probenahme von Blut bei einer Person vorgesehen, wobei das Blutentnahmeröhrchen durch ein Spritzgussverfahren unter Verwendung von homogenisiertem, recyceltem Kunststoffgranulat gemäß vorhergehender Beschreibung hergestellt ist

Zum besseren Verständnis der Erfindung wird diese anhand der nachfolgenden Figuren näher erläutert.

Es zeigen jeweils in stark vereinfachter, schematischer Darstellung:

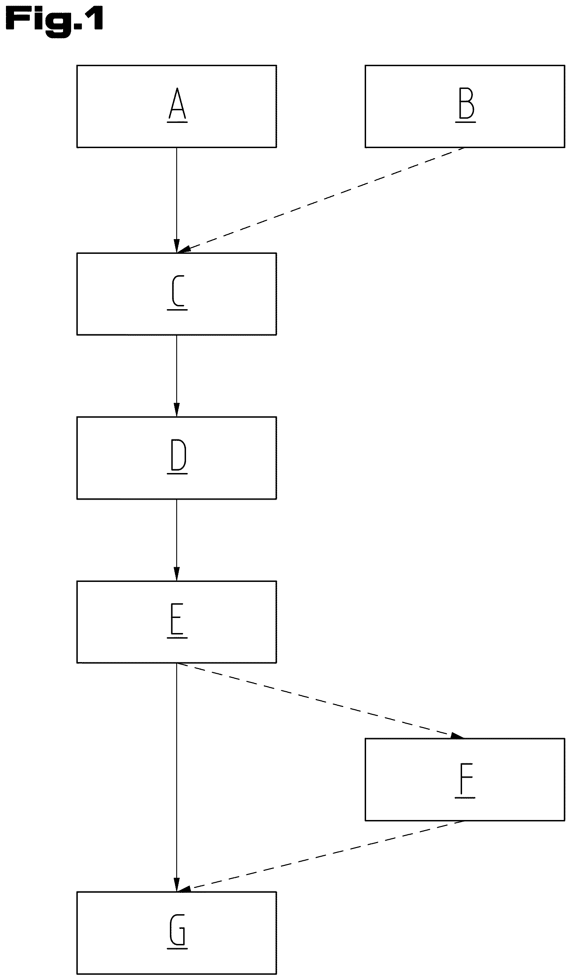

- Fig. 1: eine mögliche Ausführungsvariante des erfindungsgemäßen Verfahrens.

Wie aus Fig. 1 ersichtlich, kann das erfindungsgemäße Verfahren bzw. ein möglicher Ablauf desselben schematisch dargestellt werden.

Das Verfahren ist zur Wiederverwertung von medizinischen Kunststoffabfällen aus Blutentnahmeröhrchen, insbesondere aus Blutentnahmeröhrchen mit einem darin enthaltenen Trenngel aus PET, in einem Recyclingprozess geeignet.

Das Verfahren kann die nachfolgenden Verfahrensschritt umfassen, wobei deren Abfolge auch variierbar ist. Wenigstens umfasst es als ersten Verfahrensschritt A das sortenreine Sammeln der Blutentnahmeröhrchen in einem dafür vorgesehenen Sammelcontainer und deren Zerkleinerung. Als alternativer erster Verfahrensschritt B kann auch vorsortierter Kunststoffabfall durch ein Trennverfahren für Probenaufnahmevorrichtungen umfassend ein Blutentnahmeröhrchen und eine Verschlusskappe bereitgestellt werden. Die Einbeziehung dieser möglichen Alternative ist durch eine Strichlierte Linie im Ablaufdiagramm der Fig. 1 dargestellt. Ein derartiges Verfahren ist im Speziellen aus der beim Österreichischen Patentamt eingereichten Patentanmeldung mit Anmeldenummer A50001/2025 bekannt geworden und wird in dieser Offenbarung im, die Seiten 5 und 6 der Einreichfassung überspannenden Absatz beschrieben. Dieses Verfahren wird gemeinhin als Float-Sink-Verfahren bezeichnet und ist vorzugsweise für Probenaufnahmevorrichtungen geeignet, die in derselben Patentanmeldung beansprucht werden.

Als alternative Ausgestaltung des ersten Verfahrensschrittes A kann auch vorgesehen sein, dass die gesammelten Blutentnahmeröhrchen vorerst ausschließlich gesammelt werden, wobei diese erst bei und/oder während einer nachfolgenden ersten mikrobiologischen Aufreinigung C, wie nachfolgend beschrieben, zerkleinert werden.

Das Verfahren kann als zweiten Verfahrensschritt C eine erste mikrobiologische Aufreinigung des vorsortierten Kunststoffabfalls durch chemische Behandlung mit Peressigsäure oder einem anderen Desinfektionsmittel mit antimikrobiologischer Wirkung umfassen.

Als weiteren und dritten Verfahrensschritt D kann das Verfahren eine zweite mikrobiologische Aufreinigung durch ionisierende Strahlung, bevorzugt Röntgenbestrahlung, zur viralen Sicherheit umfassten.

In einem nachfolgenden vierten Verfahrensschritt E kann die Entfernung eines Trenngels aus dem Kunststoffabfall durch eine Kombination aus Zentrifugation, Waschung und Erwärmung des Kunststoffabfalls vorgesehen sein.

Ein fünfter und optionaler Verfahrensschritt F kann das Separieren von Etiketten und Kunststoffverunreinigungen mittels eines Flotationsverfahrens mit einem Trennmedium umfassen.

Schließlich kann das Verfahren einen sechsten und abschließenden Verfahrensschritt G umfassen, in welchem die Trocknung und Regranulierung der Kunststoffabfälle zu einem homogenisierten, recycelten Kunststoffgranulat für die Herstellung neuer Medizinprodukte vorgesehen ist.

Das erfindungsgemäße Verfahren ist insbesondere geeignet, um die hohen Reinheits- und Dekontaminationsanforderungen an Rohstoffe für die Herstellung von Gebinden für die Aufbewahrung von flüssigen, semi-soliden und festen Arzneimittelformen zu gewährleisten.

Die Ausführungsbeispiele zeigen mögliche Ausführungsvarianten, wobei an dieser Stelle bemerkt sei, dass die Erfindung nicht auf die speziell dargestellten Ausführungsvarianten derselben eingeschränkt ist.

Der Schutzbereich ist durch die Ansprüche bestimmt. Die Beschreibung und die Zeichnung sind jedoch zur Auslegung der Ansprüche heranzuziehen. Einzelmerkmale oder Merkmalskombinationen aus den gezeigten und beschriebenen unterschiedlichen Ausführungsbeispielen können für sich eigenständige erfinderische Lösungen darstellen. Die den eigenständigen erfinderischen Lösungen zugrundeliegende Aufgabe kann der Beschreibung entnommen werden.

### Bezugszeichenaufstellung

- A.: Erster Verfahrensschritt
- B.: Alternativer erster Verfahrensschritt
- C.: Zweiter Verfahrensschritt
- D.: Dritter Verfahrensschritt
- E.: Vierter Verfahrensschritt
- F.: Fünfter Verfahrensschritt
- G.: Sechster Verfahrensschritt

## Patentansprüche

1. Verfahren zur Wiederverwertung von medizinischen Kunststoffabfällen aus Blutentnahmeröhrchen, insbesondere aus Blutentnahmeröhrchen aus PET mit einem darin enthaltenen Trenngel, in einem Recyclingprozess, umfassend die Schritte:
- sammeln der Blutentnahmeröhrchen in einem dafür vorgesehenen Sammelcontainer und zerkleinern der Blutentnahmeröhrchen zu Kunststoffabfall oder
- alternatives Bereitstellen von vorsortiertem Kunststoffabfall durch ein Trennverfahren für Probenaufnahmevorrichtungen umfassend ein Blutentnahmeröhrchen und eine Verschlusskappe;
- erste mikrobiologische Aufreinigung des vorsortierten Kunststoffabfalls durch chemische Behandlung mit Peressigsäure oder einem anderen Desinfektionsmittel mit antimikrobiologischer Wirkung;
- zweite mikrobiologische Aufreinigung durch ionisierende Strahlung, bevorzugt Röntgenbestrahlung, zur viralen Sicherheit;
- Entfernung eines Trenngels aus dem Kunststoffabfall durch eine Kombination aus Zentrifugation, Waschung und Erwärmung des Kunststoffabfalls;
- optionales Separierung von Etiketten und Kunststoffverunreinigungen mittels eines Flotationsverfahrens mit einem Trennmedium;
- Trocknung und Regranulierung der Kunststoffabfälle zu einem homogenisierten, recycelten Kunststoffgranulat.

2. Verfahren nach Anspruch 1, umfassend den Schritt einer digitalen Rückverfolgbarkeit des Recyclingprozesses durch Codierung des Sammelcontainers und Verknüpfung der Codierung mit einem digitalen System, wobei das recycelte Kunststoffgranulat zur Herstellung neuer Blutentnahmeröhrchen mit, im digitalen System dokumentierter Reinheit und nachvollziehbarem Recycling verwendet wird.

3. Verfahren nach einem der vorhergehenden Ansprüche, **dadurch gekennzeichnet, dass** die Sammlung der Röhrchen mittels dem Sammelcontainer mit automatisierter Entkappung und Sortierung der Blutentnahmeröhrchen erfolgt und der Sammelcontainer mit einem Originalitätsverschluss versehen ist.

4. Verfahren nach einem der vorhergehenden Ansprüche, **dadurch gekennzeichnet, dass** die ionisierende Strahlung mit einer Dosis zwischen 10 kGy und 50 kGy durchgeführt wird, um DNA- und RNA-basierte Kontaminationen zu eliminieren.

5. Verfahren nach einem der vorhergehenden Ansprüche, **dadurch gekennzeichnet, dass** das Trenngel durch eine zusätzliche Waschung mit Wasser während der Zentrifugation und bei einer Temperatur aus einem Temperaturbereich umfassend 35°C bis 95°C entfernt wird.

6. Verfahren nach einem der vorhergehenden Ansprüche, **dadurch gekennzeichnet, dass** das Flotationsverfahren mit einer Dichteanpassung des Trennmediums erfolgt, um eine verbesserte Separierung von Fremdstoffen zu ermöglichen.

7. Verfahren nach einem der vorhergehenden Ansprüche, **dadurch gekennzeichnet, dass** die Trocknung der Kunststoffabfälle unter Vakuum erfolgt, um Restverunreinigungen zu minimieren.

8. Verfahren nach einem der vorhergehenden Ansprüche, **dadurch gekennzeichnet, dass** die Regranulierung durch eine Schmelzfiltration mittels eines Filters mit einer Maschenweite unter 50 µm, im Speziellen unter 30 µm, insbesondere unter 20 µm ergänzt wird.

9. Verfahren nach einem der vorhergehenden Ansprüche, **dadurch gekennzeichnet, dass** das recycelte Kunststoffgranulat für die erneute Herstellung von Blutentnahmeröhrchen mit einem IV-Wert zwischen 0,55 dl/g und 0,65 dl/g bereitgestellt wird.

10. Verfahren nach einem der vorhergehenden Ansprüche, wobei neue Blutentnahmeröhrchen aus dem homogenisierten, recycelten Kunststoffgranulat hergestellt werden.

11. Verwendung des, gemäß dem Verfahren nach einem der vorhergehenden Ansprüche hergestellten homogenisierten, recycelten Kunststoffgranulats zur Herstellung von neuen Medizinprodukten, insbesondere zur Herstellung von Verpackungen mit Anforderungen bezüglich der mikrobiologischen Reinheit deren Ausgangsmaterialien, im Speziellen von Pharmaverpackungen oder von Blutentnahmeröhrchen.

12. Verwendung nach Anspruch 10, **dadurch gekennzeichnet, dass** die Herstellung der neuer Medizinprodukte aus recyceltem Kunststoffgranulat mittels eines Co-Injektionsspritzgussverfahrens oder mittels eines Overmolding-Spritzgussverfahrens durchgeführt wird.

13. Blutentnahmeröhrchen zur Probenahme von Blut bei einer Person, wobei das Blutentnahmeröhrchen durch ein Spritzgussverfahren unter Verwendung von homogenisiertem, recyceltem Kunststoffgranulat gemäß einem der Ansprüche 1 bis 9 hergestellt ist.

14. Aufbewahrungsgebinde für flüssige, semi-solide und/oder feste Arzneimittel, wobei das Aufnahmegebinde durch ein Spritzgussverfahren unter Verwendung von homogenisiertem, recyceltem Kunststoffgranulat gemäß einem der Ansprüche 1 bis 9 hergestellt ist.
